# EUROPÄISCHE PATENTANMELDUNG

(11) **EP 4 679 438 A1**
(43) Veröffentlichungstag der Anmeldung: **14.01.2026**
(21) Anmeldenummer: 24187124.3
(22) Anmeldetag: 08.07.2024
(51) Int. Cl.: G16H 20/17, G16H 40/20, G16H 40/63, G16H 40/67

(54) **VERFAHREN UND SYSTEM ZUR AUTOMATISCHEN FERNPROGRAMMIERUNG VON INFUSIONSPUMPEN**

(71) Anmelder: B. Braun Melsungen AG, 34212 Melsungen (DE)
(72) Erfinder: Kever, Felix, 34125 Kassel (DE); Schmoll, Horst, 34302 Guxhagen (DE)
(74) Vertreter: Tergau & Walkenhorst Intellectual Property GmbH

(57) **Zusammenfassung**

Hauptanspruch: Verfahren zur automatischen Fernprogrammierung von Infusionspumpen (IP) mittels Infusionsaufträgen (IO), die in/von einem Patientendaten Management-System (EMR) einer medizinischen Einrichtung (ME) bereitgestellt werden, wobei die medizinische Einrichtung (ME)
• eine Anzahl von Betten aufweist, die jeweils mit einem Patienten belegbar sind,
• eine Anzahl von Infusionspumpen (IP) aufweist,
wobei
• das Patientendaten Management-System (EMR) in einem Infusionsauftrags-Backlog (IOB) Infusionsaufträge (IO) zwischenspeichert,
• die jeweilige Infusionspumpe (IP) über eine Anzahl von elektronischen Schnittstellen (SS 1, SS 2, SS3, SS 4) mit dem Infusionsauftrags-Backlog (IOB) kommuniziert und zwischengespeicherte Infusionsaufträge (IO) abruft oder empfängt,
wobei ferner
• dem jeweiligen Infusionsauftrag (IO) ein von einem Menschen lesbarer Kurzbestätigungscode (SVC) als eindeutige Kennung unmittelbar zugeordnet und bei Übermittlungen mitgegeben wird,
• dem jeweiligen Infusionsauftrag (IO) ein Medikationscontainer (MC) zugeordnet wird, der mit einem den zugeordneten Kurzbestätigungscode (SVC) enthaltenden Informationsträger ausgestattet oder verbunden ist,
• die jeweilige Infusionspumpe (IP) eine Aufnahme (10) oder einen Anschluss für einen der Medikationscontainer (MC) aufweist,
• der jeweiligen Infusionspumpe (IP) ein Eingabemittel, insbesondere ein an der Infusionspumpe (IP) angebrachtes Eingabefeld (60), zur Eingabe des zugehörigen, dem Informationsträger des Medikationscontainers (MC) entnehmbaren Kurzbestätigungscodes (SVC) zugeordnet ist,
wobei schließlich
in einem Verifikationsschritt ein am Eingabemittel eingegebener Kurzbestätigungscode (SVC) mit dem Kurzbestätigungscode (SVC), der dem von der Infusionspumpe (IP) empfangenen Infusionsauftrag (IO) unmittelbar zugeordnet ist, verglichen wird, und wobei nur bei Übereinstimmung der Kurzbestätigungscodes (SVC) der Infusionsauftrag (IO) an die Infusionspumpe (IP) übertragen und von dieser ausgeführt wird oder auf Aufforderung ausführbar ist.

## Beschreibung

Die Erfindung betrifft ein Verfahren und ein System zur automatischen Fernprogrammierung von Infusionspumpen mittels Infusionsaufträgen, die in/von einem Patientendaten Management-System einer medizinischen Einrichtung bereitgestellt werden.

Stand der Technik ist der in Teilen der Welt schon seit vielen Jahren eingesetzte AutoProgramming- oder BCMA-Workflow, wobei BCMA für Barcode Medication Administration steht, also in etwa Barcode-gestützte Medikamentengabe.

Vorausgesetzt wird ein Electronic Medical Record (EMR) System oder auch Patientendaten Management-System in einer medizinischen Einrichtung wie beispielsweise einem Krankenhaus, einem Pflegeheim oder dergleichen. Ausgehend von einem im EMR-System erstellten Medikationsauftrag für einen bestimmten Patienten, mit einer bestimmten Medikation sowie genauen Vorgaben zur Applikation werden von diesem System zunächst maschinenlesbare (Barcode-) Labels für den Medikationscontainer sowie - falls nicht bereits vorhanden - den Patienten erstellt.

Am Bettplatz des Patienten scannt die Krankenschwester das mit Barcode versehene Patientenarmband, den in der Vorbereitung mit dem entsprechenden Barcode versehenen Medikationsbehälter und zuletzt die ebenfalls mit einem Barcode versehene Infusionspumpe, welche zum Einsatz kommen soll, mit einem Scanner, welcher mit dem EMR-System verbunden ist. Dort werden die vor Ort gescannte Patienten-ID und Medikations-ID nun mit dem vorliegenden Medikationsauftrag abgeglichen (richtiger Patient? richtige Medikation? etc.) und bei erfolgreicher Prüfung an die zuvor ebenfalls gescannte Infusionspumpe gesendet.

Auf der Pumpe findet optional eine weitere Prüfung statt, indem die Medikation und die Vorgaben zur Applikation (Dosis- oder Flussrate, zu infundierendes Volumen, Limits, ...) mit den in der Medikamentenbibliothek der Infusionspumpe gespeicherten Daten und Vorgaben abgeglichen werden.

Schließlich zeigt die Infusionspumpe die empfangenen Daten zur finalen Bestätigung auf dem Display an; danach kann die Infusionstherapie gestartet werden. Die laufenden Infusionsdaten werden dann inklusive der Auftrags-ID zurück an das EMR-System gesendet, so dass sie automatisch dem entsprechenden Medikationsauftrag und/oder der elektronischen Patientenakte des Patienten zugeordnet werden können.

Die Nachteile dieses Verfahrens lassen sich wie folgt zusammenfassen:
- Ein Barcodescanner sowie eine Benutzerschnittstelle zum EMR-System müssen für jede zu startende Infusionstherapie am Bettplatz verfügbar und betriebsbereit sein oder von jeder Krankenschwester mitgeführt werden.
- Das Durchlaufen der jeweils nötigen Scanvorgänge am Patientenbett sowie der jeweiligen Bestätigungen im EMR-System ist allein schon vom Ablauf her zeitaufwändig.
- Durch die physischen Abstände zwischen der Benutzerschnittstelle des EMR-Systems, dem Patientenarmband, dem Medikationsbehälter und der Infusionspumpe ist unter Umständen ein mehrfacher räumlicher Wechsel erforderlich, welcher den Arbeitsablauf verkompliziert und weiteren Zeitaufwand verursacht.
- Die oben genannten Punkte führen zu einer geringen Akzeptanz sowohl bei den Workflow-Verantwortlichen als auch bei den Krankenschwestern / dem Pflegepersonal, insbesondere wenn es um eine Neueinführung des BCMA-Workflows geht.
- Der wesentliche Teil des BCMA-Workflows (inklusive der Scanner-basierten Verifikation des Infusionsauftrags) ist auf Seiten des EMR-Systems zu implementieren; das Infusionspumpen-System erhält lediglich den fertig geprüften Infusionsauftrag zur Anzeige auf / Ausführung durch die Pumpe. Dies hat dazu geführt, dass aktuell lediglich wenige EMR-Systeme den BCMA-Workflow unterstützen.
- Das alles kann letztlich resultieren im Auftreten von Medikations- und Behandlungsfehlern zu Lasten von Patienten, die mit dem BCMA-Workflow sicher hätten vermieden werden können.

Aufgabe der Erfindung ist es, einen Workflow (Verfahren) und ein zugehöriges System (Vorrichtung) zur einfachen, gegen Verwechslungen abgesicherten Fernprogrammierung von Infusionspumpen durch ein EMR-System bereitzustellen, der/das die Nachteile des Standes der Technik zumindest teilweise vermeidet oder abmildert und insbesondere ohne aufwendige Scanvorgänge durch das Pflegepersonal am Patientenbett auskommt.

Die genannte Aufgabe wird erfindungsgemäß gelöst durch ein Verfahren nach den Merkmalen des Anspruchs 1 sowie durch ein System (Vorrichtung) mit den Merkmalen des Anspruchs 10.

Demnach ist ein Verfahren zur automatischen Fernprogrammierung von Infusionspumpen vorgesehen mittels Infusionsaufträgen, die in/von einem Patientendaten Management-System einer medizinischen Einrichtung bereitgestellt werden, wobei die medizinische Einrichtung
- eine Anzahl von Betten aufweist, die jeweils mit einem Patienten belegbar sind,
- eine Anzahl von Infusionspumpen aufweist,
wobei
- das Patientendaten Management-System in einem Infusionsauftrags-Backlog Infusionsaufträge zwischenspeichert,
- die jeweilige Infusionspumpe über eine Anzahl von elektronischen Schnittstellen mit dem Infusionsauftrags-Backlog kommuniziert und zwischengespeicherte Infusionsaufträge abruft oder empfängt,
wobei ferner
- dem jeweiligen Infusionsauftrag ein von einem Menschen lesbarer Kurzbestätigungscode als eindeutige Kennung unmittelbar zugeordnet und bei Übermittlungen mitgegeben wird,
- dem jeweiligen Infusionsauftrag ein Medikationscontainer zugeordnet wird, der mit einem den zugeordneten Kurzbestätigungscode enthaltenden Informationsträger ausgestattet ist,
- die jeweilige Infusionspumpe eine Aufnahme oder einen Anschluss für einen der Medikationscontainer aufweist,
- der jeweiligen Infusionspumpe ein Eingabemittel zur Eingabe des zugehörigen, dem Informationsträger des Medikationscontainers entnehmbaren Kurzbestätigungscodes zugeordnet ist,
wobei schließlich
in einem Verifikationsschritt ein am Eingabemittel eingegebener Kurzbestätigungscode mit dem Kurzbestätigungscode, der dem von der Infusionspumpe empfangenen Infusionsauftrag unmittelbar zugeordnet ist, verglichen wird, und wobei nur bei Übereinstimmung der Kurzbestätigungscodes der Infusionsauftrag an die Infusionspumpe übertragen und von dieser ausgeführt wird oder auf Aufforderung ausführbar ist.

Der Begriff "Bett" oder "Bettplatz" ist in der vorliegenden Anmeldung vorzugsweise in einem weiten Sinne zu verstehen und beinhaltet jeglichen Infusionsplatz, an dem einem Patienten oder Kunden eine Infusion verabreicht werden kann.

Damit steht ein deutlich vereinfachter Workflow zur abgesicherten Fernprogrammierung von Infusionspumpen durch ein EMR-System zur Verfügung. Durch den Wegfall von bis zu drei Scan-Vorgängen und die Verlagerung sämtlicher Nutzerinteraktion auf die Infusionspumpe wird der gesamte Ablauf wesentlich schneller, nutzerfreundlicher und ist zudem leichter zu implementieren - unter Beibehaltung sämtlicher Benefits des klassischen AutoProgramming/BCMA-Workflows, insbesondere unter Ausschluss von Fehlzuordnungen der Medikation zum Patient.

In einer ersten bevorzugten Variante erfolgt eine Zuordnung zwischen Patient und Infusionspumpe indirekt über die Bettplatzzuordnung der Infusionspumpe. In einer zweiten bevorzugten Variante erfolgt eine Zuordnung zwischen Patient und Infusionspumpe über eine der Infusionspumpe bekannte oder an ihr einzugebende Patienten-ID.

Besonders vorteilhaft ist es bei beiden Varianten, wenn die Zuordnung zwischen Patient und Infusionspumpe anhand des dem Kurzbestätigungscode zugeordneten Infusionsauftrags automatisch verifiziert wird. Diese Verifikation kann bevorzugt im Patientendaten Management-System (EMR), in der Infusionspumpe oder in einem Backend eines Infusionspumpensystems erfolgen.

Eine bevorzugte Implementierung der Patienten-ID-basierten Zuordnung umfasst die zusätzliche Eingabe eines zweiten, patientenbezogenen Codes an der Pumpe (d. h. eines bevorzugt ebenfalls durch alphanumerische Codierung stark verkürzten Codes), welcher vom EMR-System erstellt und verwaltet wird und dem Patienten temporär zugewiesen wird. Dieser Code wäre entweder zusätzlich auf dem Patientenarmband aufgedruckt oder würde die üblicherweise recht lange und rein numerische Patienten-ID auf dem Armband für den genannten Zweck ersetzen. In der Konsequenz wäre dies wäre ein zusätzlicher Eingabeschritt der Pflegekraft, andererseits entfällt dabei die Notwendigkeit der Bettplatzzuordnung der Pumpe.

Um Missverständnisse auszuschließen: Die Patienten-ID wird natürlich weiterhin benötigt, um den Patienten dauerhaft eindeutig identifizieren zu können. Aber ebenso, wie er temporär ein Bett zugewiesen bekommt, wird ihm auch temporär ein Patienten-SVC zugeordnet. Beide Zuordnungen werden vom Krankenhaus verwaltet, wobei statt dem EMR-System hier auch das übergeordnete KIS (Krankenhausinformationssystem) zuständig sein kann.

In einer vorteilhaften Variante wird dem jeweiligen Infusionsauftrag ein Gültigkeitszeitraum oder eine Gültigkeitsdauer zugeordnet und im Verifikationsschritt berücksichtigt. Dadurch kann auch bei einer vergleichsweise großen medizinischen Einrichtung mit vielen Infusionen ein relativ kurzer Kurzbestätigungsode ausreichend sein.

Wenn der Kurzbestätigungsode ein alphanumerischer Code ist, ist er vom Pflegepersonal besonders einfach zu handhaben und über das Eingabefeld der Infusionspumpe einzugeben. Der Begriff "alphanumerisch" soll vorliegend sowohl die Möglichkeit eines rein numerischen Codes (insbesondere aus den Ziffern 0 bis 9) als auch eines rein buchstabenbasieren Codes (vorzugsweise aus den lateinischen Großbuchstaben A bis Z, ggf. ohne O und/oder ohne I) als auch eines aus den genannten Bestandteilen gemischten bzw. kombinierten Codes beinhalten. Ein dreistelliger alphanumerischer Code kann im zuletzt genannten Fall rund 42.000 Varianten abbilden und ist damit - auch mit Blick auf die begrenzte Gültigkeitsdauer - in aller Regel ausreichend.

Anstelle oder zusätzlich zu einer Eingabe über ein Eingabefeld / Touchdisplay an der Infusionspumpe kann eine Eingabe des Codes über ein der Infusionspumpe zugeordnetes Eingabemittel, z. B. ein Eingabefeld am Rack, oder über ein Mobile Device (Smartphone, Tablet oder dergleichen) erfolgen.

In einem bevorzugten einfachen Fall handelt es sich bei dem Informationsträger um eine ablesbare Beschriftung, insbesondere eine aufgedruckte Beschriftung, beispielsweise in Gestalt eines mit dem Kurzbestätigungscode beschrifteten Aufklebers, der auf den Medikationscontainer geklebt wird, oder in Gestalt eines beschrifteten Zettels, Streifens, Labels oder sonstigen beschrifteten Mediums, der/das mit dem Medikationsträger verbunden ist. Es ist aber auch denkbar, dass der Informationsträger den Kurzbestätigungscode in anderer Form enthält und beispielsweis maschinell / automatisiert ausgelesen um dem Eingabemittel zugeführt wird.

Die für das Verfahren genannten Merkmale und Vorteile übertragen sich analog auf die Vorrichtung.

Das im Rahmen der vorliegenden Erfindung beschriebene Systemkonzept / der Workflow der verifizierten Verwaltung von Infusionsvorgängen oder auch "Simple and Verified Infusion Administration (SVIA) erfüllt den gleichen Zweck wie der bekannte BCMA-Workflow, ist jedoch wesentlich nutzerfreundlicher und damit einfacher in der Implementierung sowie der täglichen Anwendung.

Die wesentlichen Unterschiede sind die Steuerung des gesamten Prozesses über die Infusionspumpe sowie der Wegfall sämtlicher Scanvorgänge - einerseits durch Nutzung bereits vorhandener Daten, andererseits durch Einsatz eines kurzen, von Menschen handhabbaren Kurzbestätigungscodes oder Verifizierungscodes. Das SVIA-Konzept erfordert dabei einige Neuerungen sowohl auf Seiten des EMR-Systems als auch auf Seiten des Infusionspumpen-Systems.

### Vereinfachtes Systemkonzept SVIA

Bei dem Systemkonzept "Simple and Verified Infusion Administration" (SVIA) handelt es sich um eine bedeutende Vereinfachung gegenüber dem bislang bekannten BCMA-Workflow. Zudem bietet es wesentlich mehr Flexibilität in der automatischen, technischen Verifikation der Patientenzuordnung der Infusionspumpen (sowohl indirekte Zuordnung über den Bettplatz als auch direkte Zuordnung über die Patienten-ID) und ermöglicht eine Überschreib- (Override-) Funktion durch den Benutzer (User) direkt an der Pumpe. Darüber hinaus kann das SVIA-Verfahren auch ohne die automatische, technische Verifikation der Patientenzuordnung genutzt werden, indem die korrekte Patientenzuordnung im Rahmen der finalen Freigabe durch den User bestätigt wird.

Verglichen mit anderen Verfahren ist auf Seiten des EMR-Systems ein geringfügig größerer Implementierungsaufwand erforderlich, dieser ist aber weiterhin deutlich geringer als beim klassischen BCMA-Verfahren.

### SVIA-bedingte Neuerungen

Die SVIA-bedingten Neuerungen sind insbesondere folgende:
- Statt klassischer Barcodes (eindimensional) oder QR-Codes (zweidimensional) kommt ein Infusion Order-spezifischer, menschenlesbarer und bevorzugt alphanumerischer Kurzbestätigungscode oder Short Verification Code (SVC) zur Anwendung, welcher von der Pflegekraft einfach vom Label des Medikationscontainers abgelesen und über das User Interface der Pumpe eingegeben werden kann.
- Mit bevorzugt drei alphanumerischen Zeichen (lateinische Großbuchstaben ohne "O" sowie Ziffern von 0 bis 9) bietet der SVC bereits rund 42.000 Codevarianten, was in Kombination mit einer begrenzten Gültigkeitsdauer ausreicht, um für jede Infusion Order eine eindeutigen Code erstellen zu können. Alternativ kann der dreistellige SVC auch lediglich aus Buchstaben bestehen, da die resultierenden rund 17.500 Codevarianten bei einer auf z. B. sieben Tage begrenzten Gültigkeitsdauer in vielen Fällen ebenfalls ausreichen werden, um sämtliche Infusionsaufträge eindeutig zu identifizieren. Damit kann bei der Verifikation des Medikationscontainers auf einen Scanner oder anderweitiges Lesegerät (z. B. Smart Device) verzichtet werden.

### SVIA-bedingte Neuerungen auf Seiten des EMR-Systems

- Anstatt - wie im BCMA-Workflow - jede Infusion Order nach dem Scannen und Prüfen der benötigten Daten sofort an das Infusionspumpensystem zu senden, werden sämtliche Infusion Order in einem Infusion Order Backlog (IOB) gesammelt und durch das EMR-System zum Abruf bereitgehalten.
- Dabei wird jeder Infusion Order zusätzlich zu ihrer internen, fortlaufenden und eindeutigen ID ein Order-spezifischer SVC temporär zugeordnet.
- Dieser Order-SVC wird entweder zusammen mit dem Start- und/oder Enddatum seiner aktuellen Zuordnung als zusätzliches Label auf den Medikationscontainer geklebt, oder das anderweitig vorgegebene Medication Label wird um den Order-SVC und das genannte Datum ergänzt.
- Optional wird für die Infusion Order eine früheste Startzeit und/oder eine begrenzte zeitliche Gültigkeit festgelegt, welche dann Einfluss auf die Beantwortung von Order-SCV-basierten Anfragen einer Infusionspumpe durch das EMR-System hat.
- Denkbar ist auch eine räumliche bzw. logische Einschränkung, z. B. auf eine bestimmte Care Unit oder einen bestimmten Nutzerkreis (diensthabende Schwester, Schwestern der jeweiligen Station etc.). In diesem Fall müssen zum einen die erlaubten Ausprägungen der zusätzlichen Parameter ebenfalls in der Infusion Order enthalten sein, zum anderen müssen die zusätzlichen Parameter zusammen mit dem Order-SVC an das EMR-System gesendet werden. (Die ID bzw. der SVC der Pflegekraft müssen zusätzlich zum Order-SVC an der Infusionspumpe eingegeben werden, die der Pumpe zugeordnete Care Unit oder die Uhrzeit stellt das Infusionspumpensystem bereit).
- Sobald bzw. solange das Backlog des EMR-Systems noch nicht zur Ausführung gebrachte Infusionsaufträge enthält, ist das EMR-System empfangsbereit für eingehende Anfragen vom Infusionspumpensystem (bzw. den darin vorhandenen Infusionspumpen).
- Basierend auf Order-SVCs versucht das EMR-System, eingehende Anfragen einem vorhandenen, noch nicht zur Ausführung gebrachten Infusionsauftrag zuzuordnen.
- Im Erfolgsfall sendet das EMR-System für diesen Infusionsauftrag eine klassische Infusion Order gemäß HL7/IHE Transaction Profile (PCD-03) an das Infusionspumpensystem. Dabei wird diejenige Pumpe als Empfänger eingetragen, an welcher zuvor der Order SVC eingegeben wurde.

Eine automatische Verifikation der Zuordnung zwischen Patient und Pumpe kann auf Wunsch im ERM-System erfolgen, entweder indirekt über die Bettplatzzuordnung der Pumpe oder direkt über die Patienten-ID. Im letzteren Fall wird zunächst jede Infusionspumpe einem Patienten zugeordnet. Die geschieht bevorzugt durch das sogenannte CPA-Verfahren. Dieses Verfahren erlaubt die einfache, scannerlose Zuordnung der potenziell 15- bis 18-stelligen Patienten-ID zu einem Medizingerät über einen innerhalb des EMR-Systems temporär zugeordneten Patienten-SVC. Typischerweise trägt der jeweilige Patient ein Patientenarmband, das den Patienten-SVC ablesbar enthält.
- Sofern die automatische Verifikation der Patientenzuordnung auf Basis der Patienten-ID bereits im EMR-System erfolgen soll, prüft dieses vor dem Versenden der Infusion Order zunächst, ob die hinterlegte Patienten-ID mit der von der Pumpe empfangenen Patienten-ID übereinstimmt und sendet andernfalls eine Fehlermeldung.
- Sofern die automatische Verifikation der Patientenzuordnung auf Basis des Bettplatzes bereits im EMR-System erfolgen soll, prüft dieses vor dem Versenden der Infusion Order zunächst, ob der dort hinterlegte Bettplatz mit der von der Pumpe empfangenen Bettplatz-Zuordnung übereinstimmt und sendet andernfalls eine Fehlermeldung.
- Falls die Anfrage nicht zugeordnet werden kann oder weitere, optionale Ausführungskriterien nicht erfüllt sind (Zeitfenster, festgelegte Reihenfolge mehrerer Infusionsaufträge, bestimmter Nutzerkreis, s.o.) sendet das EMR-System ebenfalls eine Fehlermeldung an das Infusionspumpensystem bzw. die anfragende Infusionspumpe.

### SVIA-bedingte Neuerungen im Infusionspumpen-System

- Zum Abruf eines bestehenden Infusionsauftrags lässt sich an jeder Infusionspumpe ein vom Medikationscontainer abgelesener Order-SVC eingeben, idealerweise über ein speziell dafür ausgelegtes Soft-Keyboard.
- Nach der Eingabe wird der Order-SVC entweder direkt oder über das Infusionspumpen-Backend an das EMR-System gesendet, um die entsprechende Infusion Order abzurufen bzw. auszulösen.
- Sofern zu dem eingegebenen Order-SVC im Backlog des EMR-Systems kein Infusionsauftrag gefunden werden kann, die (gegebenenfalls dort implementierte) Verifikation der Patientenzuordnung fehlschlägt oder weitere Prüfkriterien nicht erfüllt sind, zeigt die Infusionspumpe vom EMR-System generierte Fehlermeldungen an.
- Sofern für den Abruf der Infusion Order weitere Prüfkriterien im EMR-System herangezogen werden sollen, müssen die entsprechenden Daten ebenfalls mitgesendet werden (etwa die Care Unit-Zuordnung der Pumpe oder die ID bzw. der SVC des Users).

Eine automatische Verifikation der Zuordnung zwischen Patient und Pumpe kann - wie weiter oben erwähnt - auf Wunsch im ERM-System erfolgen, entweder indirekt über die Bettplatzzuordnung der Pumpe oder direkt über die Patienten-ID.
- Sofern die automatische Verifikation der Patientenzuordnung auf Basis der Patienten-ID im EMR-System stattfinden soll, muss die in der Pumpe gespeicherte Patienten-ID ebenfalls mitgesendet werden.
- Sofern die automatische Verifikation der Patientenzuordnung auf Basis der Bettplatz-Zuordnung im EMR-System stattfinden soll, muss die Bettplatz-Zuordnung der Pumpe ebenfalls mitgesendet werden.

Eine automatische Verifikation der Zuordnung zwischen Patient und Pumpe kann aber auch im Infusionspumpensystem erfolgen (entweder im Backend oder direkt in der Pumpe), sowohl indirekt über die Bettplatzzuordnung der Pumpe als auch direkt über die Patienten-ID:
- Sofern die die automatische Verifikation der Patientenzuordnung auf Basis der Patienten-ID im Infusionspumpen-Backend stattfinden soll, muss dort geprüft werden, ob die der Pumpe zugeordnete Patienten-ID mit der in der Infusion Order genannten Patienten-ID übereinstimmt (ggfs. Fehlermeldung an Pumpe und EMR-System).
- Sofern die die automatische Verifikation der Patientenzuordnung auf Basis der Bettplatz-Zuordnung im Infusionspumpen-Backend stattfinden soll, muss dort geprüft werden, ob die Bettplatz-Zuordnung der Pumpe und die in der Infusion Order genannte Bettplatz-Zuordnung des Patienten übereinstimmen (ggfs. Fehlermeldung an Pumpe und EMR-System).
- Sofern die die automatische Verifikation der Patientenzuordnung auf Basis der Patienten-ID in der Pumpe stattfinden soll, muss dort geprüft werden, ob die der Pumpe zugeordnete Patienten-ID mit der in der Infusion Order genannten Patienten-ID übereinstimmt (ggfs. Fehlermeldung an das EMR-System).
- Sofern die die automatische Verifikation der Patientenzuordnung auf Basis der Bettplatz-Zuordnung in der Pumpe stattfinden soll, muss dort geprüft werden, ob die Bettplatz-Zuordnung der Pumpe und die in der Infusion Order genannte Bettplatz-Zuordnung des Patienten übereinstimmen (ggfs. Fehlermeldung an das EMR-System).
- Nach erfolgreichem Empfang der Infusion Order vom EMR-System werden sämtliche Daten auf dem Display der Pumpe zur finalen Verifikation durch den Nutzer angezeigt. Andernfalls wird der Vorgang abgebrochen und eine entsprechende Fehlermeldung an das EMR-System gesendet.

### Voraussetzungen für die automatische Verifikation der Patientenzuordnung:

- Indirekte Patientenzuordnung über den Bettplatz:
   ∘ Im EMR-System sind die Patienten zu jedem Zeitpunkt eindeutig einem Bettplatz zugeordnet UND
   ∘ Im Infusionspumpensystem sind die Pumpen zu jedem Zeitpunkt eindeutig einem Bettplatz zugeordnet UND
   ∘ Das Verzeichnis der Bettplätze bzw. die für die indirekte Patientenzuordnung genutzte Hospitalstruktur in beiden Systemen ist kongruent
- ODER Direkte Patientenzuordnung über die Patienten-ID:
   o Jede Infusionspumpe lässt sich über die Patienten-ID oder die patientenbezogene Fallnummer eindeutig und sicher einem Patienten zuordnen
- Die Anzahl der möglichen Ausprägungen des gewählten SVC (etwa alphanumerisch oder rein zeichenbasiert, etwa zweistellig oder dreistellig oder vierstellig) in Kombination mit der gewählten Gültigkeitsdauer) reichen aus, um die Anzahl der innerhalb dieser Gültigkeitsdauer gleichzeitig anfallenden Infusionen sicher abzubilden.

### Option: Manuelle Verifikation von Infusionsdaten und Patientenzuordnung

Auf Wunsch kann auf die automatische Verifikation der Patientenzuordnung verzichtet und die Implementierung nochmals deutlich vereinfacht werden. In diesem Fall wird lediglich der Order-SVC an der Pumpe eingegeben und - im Falle einer erfolgreichen Zuordnung im Order Backlog des EMR-Systems - die passende Order an die entsprechende Pumpe gesendet. Dort werden sämtliche Daten auf dem Display der Pumpe zur Verifikation durch den Nutzer angezeigt, eine vorherige, automatische Verifikation der Patientenzuordnung (sei es in der Pumpe, im Pumpen-Backend oder im EMR-System) findet nicht statt. Andernfalls wird der Vorgang abgebrochen und eine entsprechende Fehlermeldung an das EMR-System gesendet.

### Beispielhafter Ablauf der SVIA-basierten Programmierung einer Infusionspumpe

Ein möglicher Ablauf der SVIA-basierten Programmierung einer Infusionspumpe wird nachfolgend rein beispielhaft beschrieben, und zwar für die Variante: Patientenzuordnung über die Patienten-ID, automatische Verifikation in der Pumpe.
1. Im EMR-System wird die Infusion Order für den Patienten X angelegt und im Backlog gespeichert.
2. In der Krankenhausapotheke oder auf der Station wird der entsprechende Medikationscontainer vorbereitet und mit einem Aufkleber versehen, der den Order-spezifischen SVC, dessen Gültigkeitszeitraum, den Namen des Patienten sowie den Namen der Medikation enthält.
3. Die Pflegekraft legt den Medikationscontainer in eine beliebige dem Patienten X zugeordnete Pumpe und gibt dort den Order-SVC ein.
4. Die Pumpe sendet (i.d.R. über das Infusionspumpen-Backend) eine Anfrage an das EMR-System, die mindestens der Order-SVC und die eindeutige ID der Pumpe enthält.
5. Das EMR-System findet über den Order-SVC den Infusionsauftrag in seinem Backlog und sendet die entsprechende Infusion Order an das Infusionspumpen-System (adressiert an die in der Anfrage enthaltene Pumpen-ID).
6. Über das Infusionspumpen-Backend empfängt (je nach Konfiguration ggfs. mit vorherigem Lookup der Medikation in der DrugLibrary des Infusionspumpensystems) die Pumpe die Infusion Order und prüft, ob die darin enthaltene Patienten-ID mit der in der Pumpe hinterlegten Patienten-ID übereinstimmt. Bei einer Abweichung erfolgt eine Fehlermeldung auf dem Pumpen-UI, optional mit Übersteuerungsmöglichkeit durch den Nutzer und/oder Versand einer entsprechenden Nachricht an das EMR-System.
7. Im Erfolgsfall zeigt die Pumpe sämtliche Order-Daten und Infusionsparameter auf dem lokalen User Interface zur finalen Prüfung durch die Pflegekraft (Vergleich mit den Auftragsdaten im EMR-System und/oder mit den Patienten- und Medikationsnamen auf dem Medication Container Label). Nach erteilter Freigabe kann die Infusion an der Pumpe gestartet werden,
andernfalls wird die Infusion Order abgebrochen und eine entsprechende Rückmeldung an das EMR-System gesendet.

Somit besteht der Arbeitsablauf für die Pflegekraft am Krankenbett in lediglich drei Schritten:
- Einlegen des mit SVC versehenen Medikationscontainers in eine geeignete Pumpe
- Eingabe des Order-SCVs an dieser Pumpe
- Finale Bestätigung und Freigabe der an der Pumpe angezeigten Auftragsdaten

Hinweise:
- Sofern die Pumpe noch nicht dem Patienten zugeordnet ist, sind gemäß dem oben erwähnten, bevorzugt eingesetzten CPA-Verfahren zuvor lediglich zwei Schritte einmalig erforderlich:
   - Ablesen des Patienten-SCV vom Armband und Eingabe an der Pumpe
   - Bestätigung der Patientenzuordnung anhand der auf dem Pumpendisplay angezeigten Patientendaten (Name, Alter, Geschlecht, etc.)
- Wie weiter oben bereits erwähnt ist die automatische, technische Verifikation der Patientenzuordnung beim SVIA-Verfahren jedoch optional (anders als im klassischen BCMA-Verfahren, wo sie die Voraussetzung für die Adressierung der Infusion Order ist).

### Vorteile des SVIA-Systemkonzeptes:

- Kein User Interface des EMR-Systems wird für die Programmierung der Pumpe benötigt.
- Kein Scanner oder Smart Device wird für die Programmierung der Pumpe benötigt.
- Anstatt drei Scanvorgänge an unterschiedlichen Stellen durchzuführen und über das User Interface des EMR-Systems zu steuern, muss die Pflegekraft lediglich einen einfachen, dreistelligen Code an der Pumpe eingeben.
- Sowohl aus Sicht des EMR-Systems als auch aus Sicht des Krankenhauses ist eine Implementierung wesentlich einfacher und schneller umzusetzen.
- Anders als im VIA-Konzept ist die im Infusionspumpen-Backend verortete Instanz des Infusion Order Manager (= zusätzliche Applikation) nicht erforderlich, was den Entwicklungsaufwand auf Seiten des Infusionspumpensystems deutlich reduziert.
- Das SVIA-Konzept bietet maximale Flexibilität bei der automatischen Verifikation der Patientenzuordnung (wahlweise über den Bettplatz oder über die Patienten-ID, wahlweise im EMR-System, im Infusionspumpen-Backend oder in der Pumpe), auf Wunsch kann diese aber auch weggelassen werden und manuell im Rahmen der ohnehin erforderlichen User-Freigabe erfolgen.
- Für den Fall einer fehlgeschlagenen automatischen Verifikation der Patientenzuordnung bietet das SVIA-Konzept die Möglichkeit einer Override-Funktion durch den User (z. B. wenn die Bettplatzzuordnung des Patienten im EMR-System nicht aktuell ist, so dass die automatische Verifikation fehlschlägt).
- Das EMR-System kann für jede Infusion Order ein zulässiges Abruf-Zeitfenster oder weitere Kriterien festlegen.
- Vorgegebene Ausführungsreihenfolgen von Infusion Ordern können vom EMR-System vorgegeben und automatisch überwacht werden (z. B.: Infusion Order B lässt sich erst abrufen, wenn Infusion Order A gestartet oder abgeschlossen wurde).
- Auch das SVIA-Konzept basiert auf dem bekannten und in einigen Teilen der Welt seit Jahren etablierten AutoProgramming/BCMA-Workflow unter Nutzung von HL7-Kommunikation und IHE Transaction Profiles.

Mehrere Ausführungsbeispiele der Erfindung werden anhand der beigefügten Zeichnungen näher erläutert. Es zeigt:
FIG. 1 eine schematische Übersicht über eine medizinische Einrichtung, in der Infusionen an Patienten verabreicht werden, wobei verschiedene Prozessabläufe durch Pfeile veranschaulicht sind, und
FIG. 2 eine schematische Darstellung einer Infusionspumpe zur Verwendung in einer derartigen Einrichtung.
FIG. 3 bis 10 zeigen verschiedene Varianten einer alterativen Implementierung des in FIG 1 und 2 beschriebenen Systemkonzepts. Dabei gilt insbesondere folgende Zuordnung:

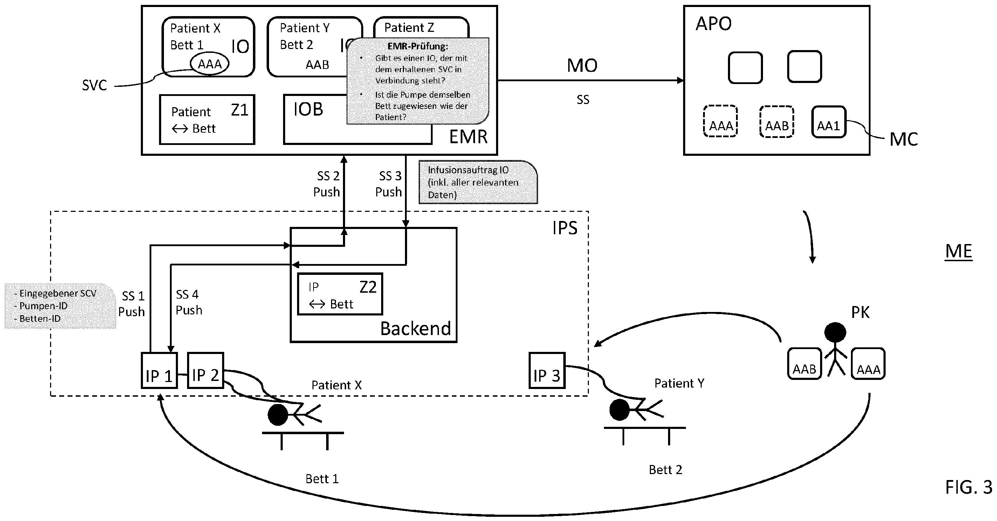

FIG. 3 Patientenzuordnung via Bettplatz, automatische Verifikation im EMR-System

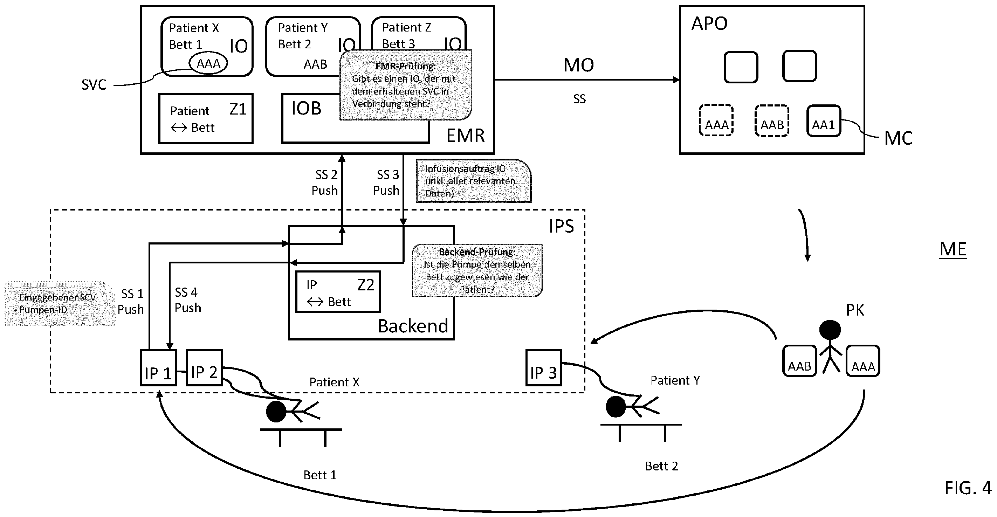

FIG. 4 Patientenzuordnung via Bettplatz, automatische Verifikation im Infusionspumpen-Backend

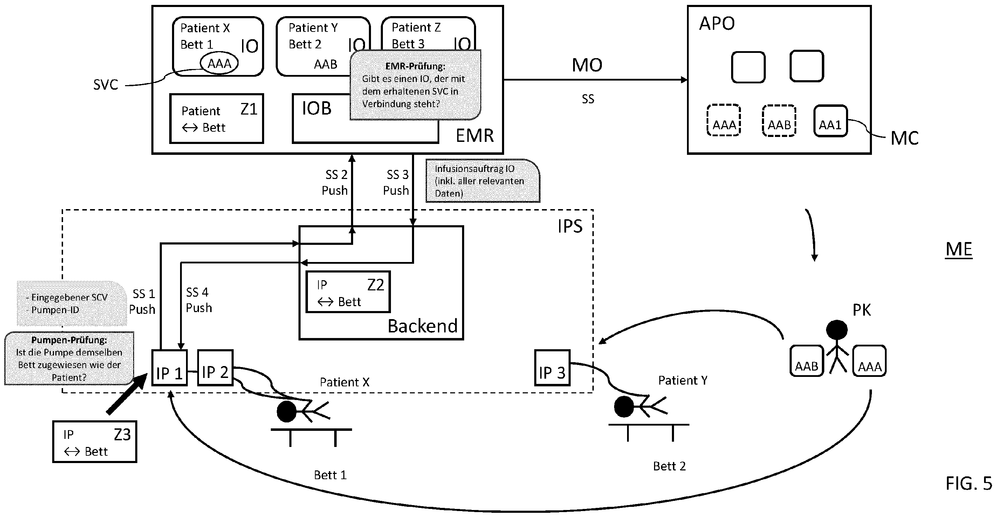

FIG. 5 Patientenzuordnung via Bettplatz, automatische Verifikation in der Infusionspumpe

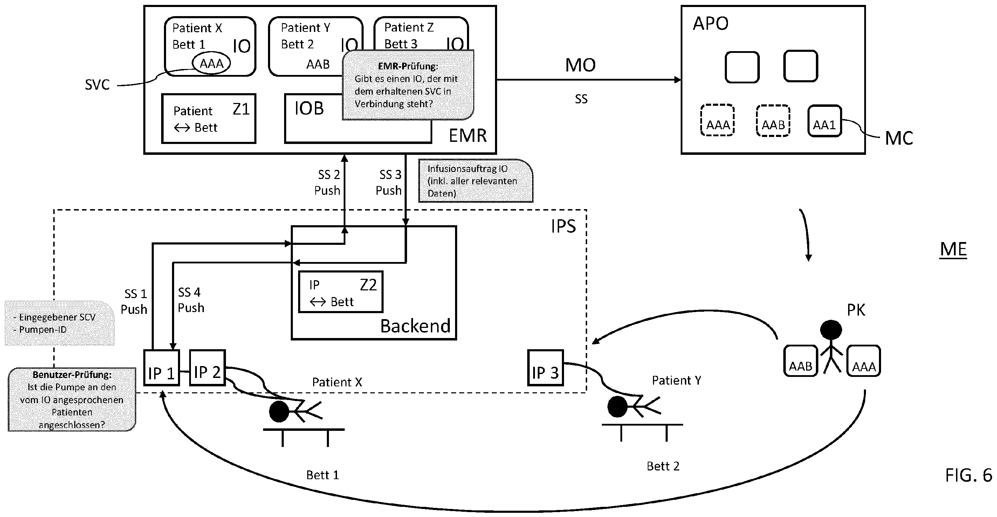

FIG. 6 Patientenzuordnung via Bettplatz, manuelle Verifikation an der Infusionspumpe

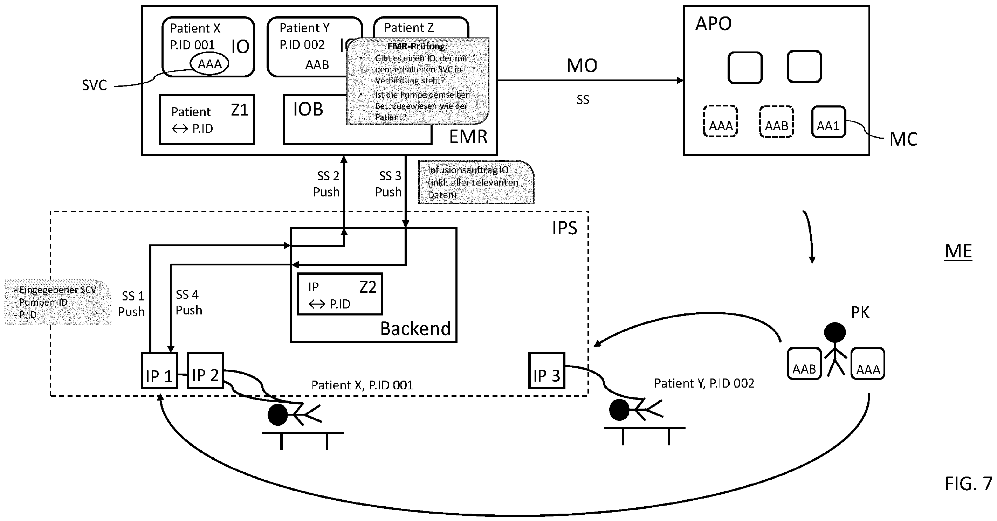

FIG. 7 Patientenzuordnung via Patienten-ID, automatische Verifikation im EMR-System

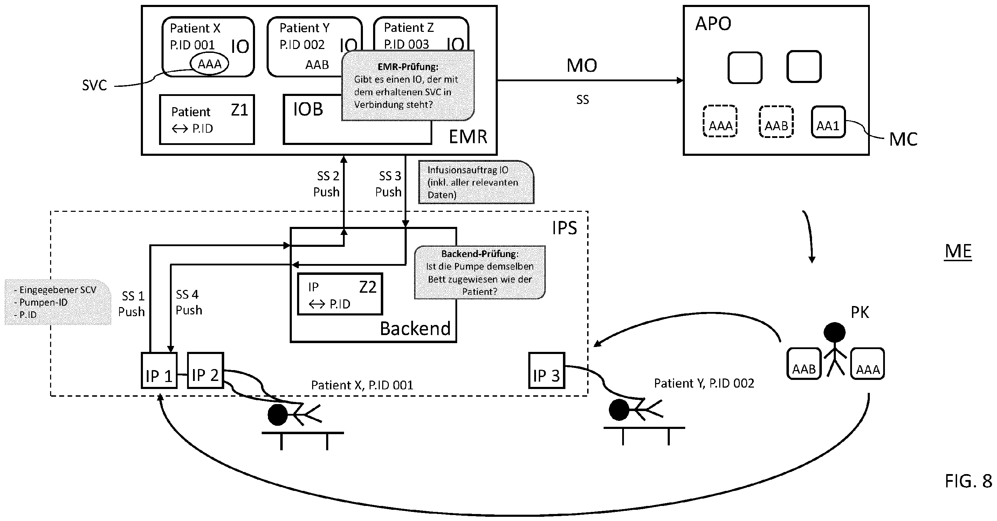

FIG. 8 Patientenzuordnung via Patienten-ID, automatische Verifikation im Infusionspumpen-Backend

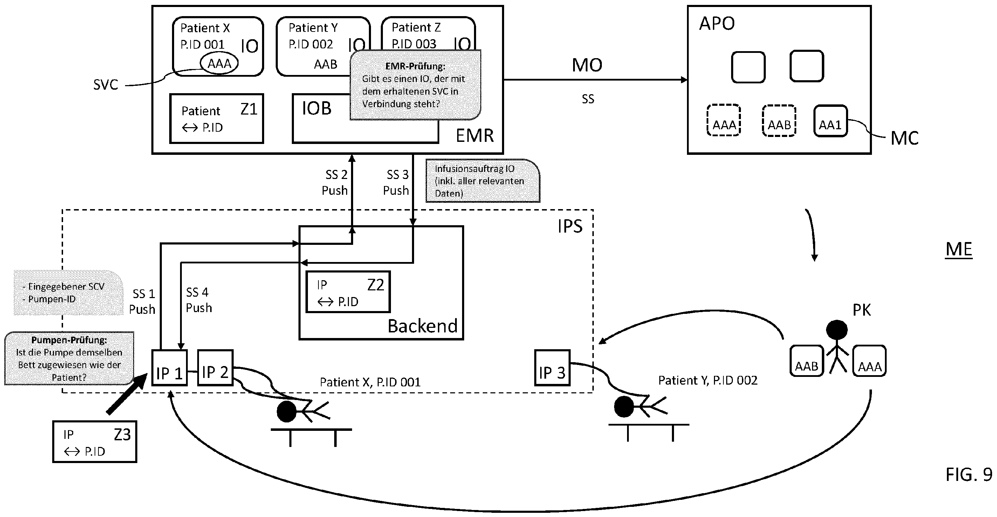

FIG. 9 Patientenzuordnung via Patienten-ID, automatische Verifikation in der Infusionspumpe

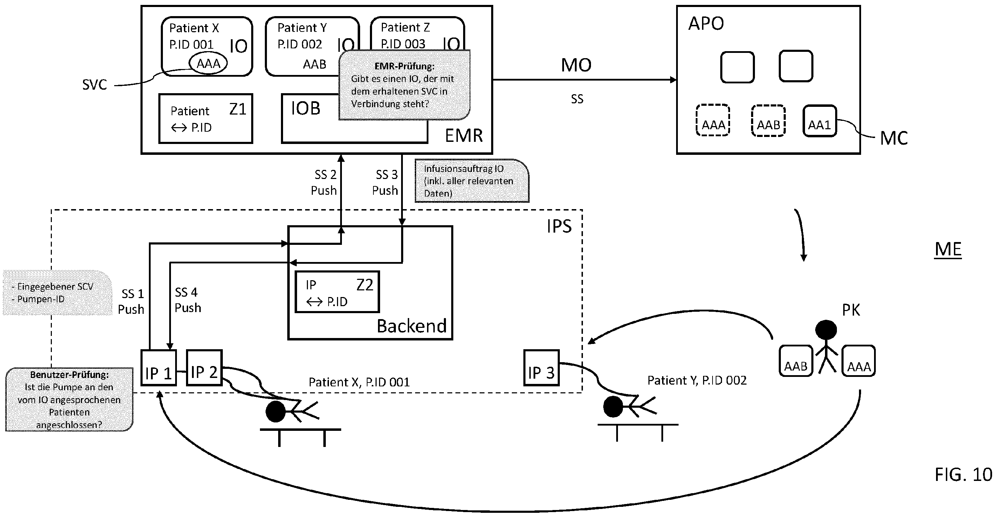

FIG. 10 Patientenzuordnung via Patienten-ID, manuelle Verifikation an der Infusionspumpe

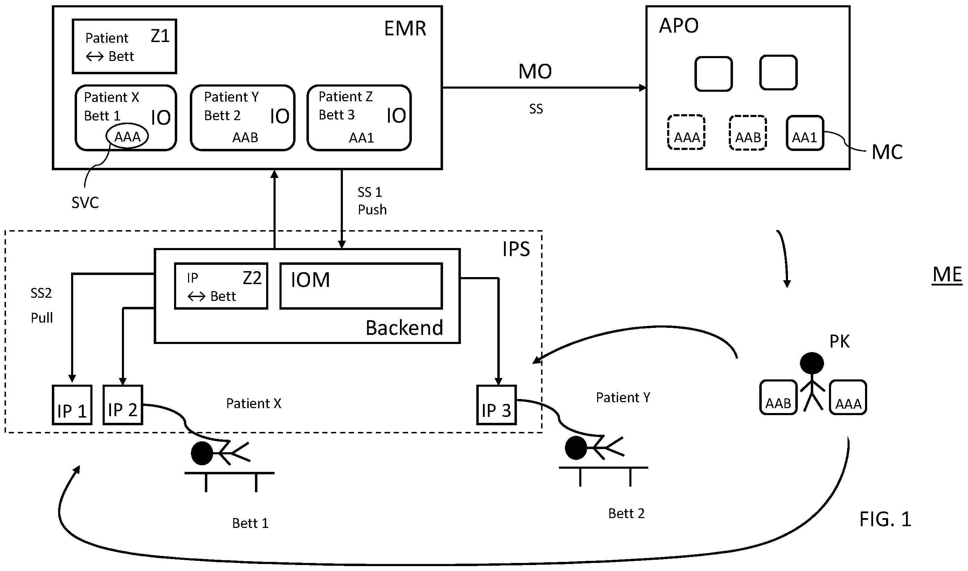

FIG. 1 gibt eine schematische Übersicht über eine medizinische Einrichtung ME, in der Infusionen an Patienten verabreicht werden. Dabei kann es sich beispielsweise um ein Krankenhaus oder ein Pflegeheim handeln. Die medizinische Einrichtung weist eine Mehrzahl von eindeutig identifizierbaren Betten auf, wobei hier im Beispiel die Identifizierung einfach über eine fortlaufende Bettnummer (Bett 1, Bett 2, ...) erfolgt. Im Allgemeinen kann die Identifizierung über eine Bettplatz-ID erfolgen. Jedes Bett kann mit einem Patienten belegt werden, der durch eine eindeutige Patienten-ID und/oder seine persönlichen Daten identifizierbar ist. Hier im Beispiel sind dies der Einfachheit halber Patient X, der Bett 1 belegt, und Patient Y, der Bett 2 belegt usw. Die aktuell gültige Zuordnung Z1 zwischen Betten und Patienten ist als Datensatz in einem elektronischen Patientendaten Management-System (engl. Electronic Medical Record-System) EMR hinterlegt.

Jedem der Betten ist zu einem beliebigen Zeitpunkt eine Anzahl von Infusionspumpen IP zugeordnet, wobei die Anzahl die Werte 0, 1, 2, ... annehmen kann. Das heißt, die möglichen Fälle "keine" oder "mehrere" Infusionspumpen IP sind enthalten, wobei jede der Infusionspumpen IP genau (und maximal) einem Bett zugeordnet ist. Die Infusionspumpen IP sind anhand einer Pumpen-ID eindeutig identifizierbar, hier im Beispiel in Gestalt einer fortlaufenden Nummerierung (IP 1, IP 2, ...). Im dargestellten aktuellen Zustand sind die Infusionspumpen IP 1 und IP 2 dem Bett 1 (und damit dem Patienten X) zugeordnet, während die Infusionspumpe IP 3 dem Bett 2 (und damit dem Patienten Y) zugeordnet ist. Die aktuelle Zuordnung Z2 zwischen Betten und Infusionspumpen ist als Datensatz in einem Backend eines Infusionspumpen-Systems IPS, welches die Infusionspumpen IP 1, IP 2, ... umfasst, hinterlegt.

Für eine effiziente und sichere Durchführung anstehender Infusionen ist eine vom EMR veranlasste und gesteuerte Fernprogrammierung der zugeordneten Infusionspumpe IP für den jeweiligen spezifischen Infusionsvorgang vorgesehen. Dazu generiert das EMR nach den Vorgaben der behandelnden Mediziner eine Anzahl von Infusionsaufträgen (engl. Infusion Orders) IO. Jeder Infusionsauftrag IO ist genau einem Patienten in dem von ihm belegten Bett zugeordnet und enthält Angaben zu dem zu verabreichenden Medikament bzw. zur Infusionslösung, zur Menge, zur Flussrate usw. Optional kann der Infusionsauftrag IO mit einem Gültigkeitszeitraum bzw. einer Gültigkeitsdauer versehen sein. Auch eine Verabreichungsreihenfolge im Falle mehrerer Infusionen für denselben Patienten kann optional in den Infusionsauftrag IO integriert sein.

Des Weiteren wird jedem Infusionsauftrag IO ein eindeutiger Kurzbestätigungscode (engl. Short Verification Code) SVC zugeordnet, bei dem es sich im Beispiel um einen dreistelligen alphanumerischen Code handelt. Beispielsweise kann jede der drei Stellen gewählt sein aus der Gesamtheit der Großbuchstaben A bis Z und den Ziffern von 0 bis 9, wobei zur Vermeidung von Verwechslungen vorzugsweise auf die Verwendung von O (und ggf. auch I) verzichtet wird. Dies ergibt beispielsweise (25+10)^3 = 42.875 verschiedene Kennzeichnungsmöglichkeiten, bei einem alternativ möglichen vierstelligen Code und/oder bei Zulassung von Kleinbuchstagen noch deutlich mehr. Hier im Beispiel ist dem Infusionsauftrag IO für den Patienten X im Bett 1 der SVC "AAA" zugeordnet, während der Infusionsauftrag IO für den Patienten Y im Bett 2 den SVC "AAB" trägt. Der SVC kann jeweils in den Infusionsauftrag IO integriert oder als separates Element zugeordnet bzw. beigefügt sein.

Aus den Infusionsaufträgen IO werden Medikationsaufträge (engl. Medication Orders) MO abgeleitet oder generiert, die über eine geeignete Schnittstelle SS, vorzugsweise elektronisch, an eine (Krankenhaus-) Apotheke APO oder an einen Medikamentenlieferant übermittelt werden. Im Beispiel fertigt die Apotheke APO die benötigten Infusionslösungen gemäß den individuellen Vorgaben hinsichtlich Zusammensetzung, Menge usw. an und/oder stellt sie in Form verabreichungsbereiter Beutel, Spritzen oder dergleichen, die zusammenfassend auch als Medikationscontainer MC bezeichnet werden, bereit.

Bei der Übermittlung des Medikationsauftrags MO wird auch der zugeordnete Kurzbestätigungscode SVC übermittelt und dem jeweiligen Medikationscontainer als Beschriftung (Label) zugeordnet, beispielsweise durch Anbringung eines entsprechend bedruckten Aufklebers oder Etikettes. Im Beispiel sieht man unter anderem die mit den Kurzbestätigungscodes "AAA" und "AAB" beschrifteten Medikationscontainer MC, die in eindeutiger Weise den oben besprochen Infusionsaufträgen IO zugeordnet sind. Neben dem Kurzbestätigungscode SVC kann die Beschriftung des Medikationscontainers MC auch noch andere Angaben umfassen, etwa solche, die im zugeordneten Infusionsauftrag IO enthalten sind.

Das Patientendaten Management-System EMR übermittelt die einzelnen Infusionsaufträge IO unmittelbar nach deren Erstellung über eine geeignete elektronische Schnittstelle SS 1 mittels Push-Kommunikation an das Backend des Infusionspumpensystems IPS, wo sie in einem als Infusionsauftragsmanager (engl. Infusion Order Manager) IOM bezeichneten Programmmodul abgelegt werden. Dort stehen die übermittelten Infusionsaufträge IO zum Abruf durch die Infusionspumpen IP bereit, wie weiter unten näher erläutert wird.

Eine Pflegekraft PK oder ein sonstiger Benutzer entnimmt einer Benutzerschnittstelle des EMR beispielsweise die Information, dass für den Patienten X im Bett 1 ein Infusionsauftrag IO (mit dem Kurzbestätigungscode "AAA") bereitsteht, und entsprechend für den Patienten Y im Bett 2 (mit dem Kurzbestätigungscode "AAB"). Er/sie holt die den Infusionsaufträgen IO zugeordneten und entsprechend beschrifteten Medikationscontainer MC aus der Apotheke APO ab und bringt sie nacheinander zu den zugeordneten Betten und somit den zugeordneten Patienten. Beispielsweise weiß oder sieht die Pflegekraft, dass der Medikationscontainer MC mit dem SVC "AAA" dem Patienten X im Bett 1 zugeordnet ist. Dort stehen die beiden Infusionspumpen IP 1 und IP 2 bereit, und sofern beide nicht belegt sind, hat die Pflegekraft im Regelfall die freie Wahl zwischen beiden. Beispielsweise entscheidet sie sich für die Infusionspumpe IP 1 zur Durchführung der Infusion am Bett 1. In anderen Fällen mag die Wahlfreiheit durch eine schon vorhandene Belegung und/oder durch die Anzahl der Infusionspumpen am Bett und/oder durch weitere Kriterien eingeschränkt sein. Beispielsweise steht am Bett 2 nur die Infusionspumpe IP 3 zur Durchführung des Infusionsauftrags IO mit dem SVC "AAB" zur Verfügung.

Beispielsweise legt die Pflegekraft PK den Medikationscontainer MC mit dem SVC "AAA" in die Aufnahme der Infusionspumpe IP 1 am Bett 1 ein und aktiviert durch eine Eingabe an einem Eingabefeld (Tastatur oder Touch-Display) eine Pull-Kommunikation, bei der sich die Infusionspumpe über eine Schnittstelle SS 2 vom Backend des Infusionspumpensystems IPS einen ihr zugeordneten Infusionsauftrag IO abholt. Beispielsweise teilt dazu die Infusionspumpe IP 1 ihre Pumpen-ID dem Backend mit. Anhand der bekannten Zuordnung Z2 zwischen Infusionspumpen und Betten erkennt das Backend die Zugehörigkeit der Infusionspumpe IP 1 zum Bett 1 und überprüft das Vorhandensein von dem Bett 1 zugeordneten Infusionsaufträgen IO im Infusionsauftragsmanager IOM. Im Erfolgsfall wird der entsprechende Infusionsauftrag IO (hier mit dem SVC "AAA"), alternativ erst einmal nur eine ihm zugeordnete ID, über die elektronische Schnittstelle SS 2 vom Backend zur Infusionspumpe (hier IP 1) übermittelt.

Zur Verifikation, dass der richtige Medikationscontainer MC in die Aufnahme der Infusionspumpe IP eingelegt wurde, wird die Pflegekraft PK über ein Display an der Infusionspumpe IP aufgefordert, über das Eingabefeld den SVC, den sie vom Medikationscontainer MC abliest, einzugeben. Bei korrekter Eingabe (hier "AAA") wird die Infusionspumpe IP entsprechend dem vom Backend erhaltenen bzw. empfangenen Infusionsauftrag IO programmiert bzw. ein bereits zuvor erhaltener Infusionsauftrag IO oder eine erfolgte Programmierung wird zur Ausführung freigegeben. Andernfalls wird eine Fehlermeldung ausgegeben.

In einer Abwandlung des Verfahrens kann vorgesehen sein, dass der Benutzer direkt beim oder nach dem Einlegen des Medikationscontainers MC in die Aufnahme den zugehörigen SVC im Eingabefeld der Infusionspumpe IP eingibt und dadurch veranlasst, dass die Infusionspumpe IP beim Backend nachfragt, ob dort ein diesem SVC zugeordneter Infusionsauftrag IO hinterlegt ist. Im Erfolgsfall "zieht" sich die Infusionspumpe IP eben jenen Infusionsauftrag aus dem IOM im Backend. Die weiter oben beschriebene Auswahl aus einer Liste offener Medikationsaufträge wäre in diesem Fall jedoch nicht möglich, da die Auswahl durch Eingabe des SVC bereits erfolgt ist - insofern könnte die Liste der weiteren Aufträge für diesen Patienten hier lediglich zur Information angezeigt werden.

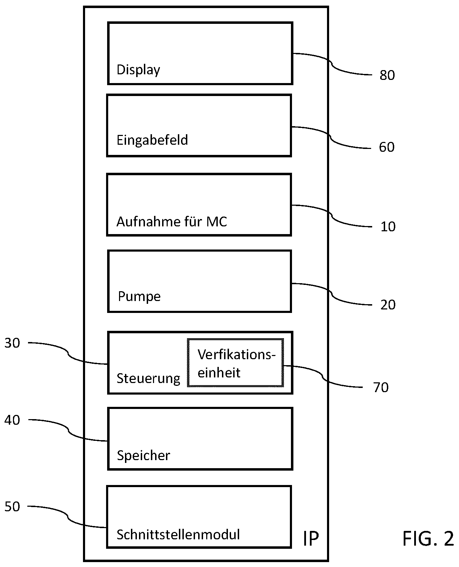

Eine Infusionspumpe IP zur Verwendung in dem beschriebenen Szenario ist in FIG. 2 schematisch dargestellt. Die Infusionspumpe IP weist eine Aufnahme 10 für einen Medikationscontainer MC auf und eine an den Medikationscontainer MC anschließbare Pumpe 20, die durch einen integrierte Steuerung 30 angesteuert wird. Ein individuell auf den Medikationscontainer MC und den Behandlungsfall abgestimmter Infusionsauftrag IO ist nach Art eines Steuerprogramms in einem beschreibbaren Speicher 40 hinterlegt bzw. durch Hineinladen hinterlegbar. Der Infusionsauftrag IO kann über ein Schnittstellenmodul 50 als Bestandteil der Schnittstelle SS 2 per "Pull" Kommunikation von einem Backend eines Infusionspumpen-systems IPS empfangen werden. Ein zugehöriger, auf dem Medikationscontainer MC aufgedruckter bzw. als Label beigefügter Kurzbestätigungscode SVC wird dazu von einer Pflegekraft PK abgelesen, über ein Eingabefeld 60 eingegeben und dann von einer Verifikationseinheit 70 der Steuerung 30 mit dem unmittelbar dem Infusionsauftrag IO zugeordneten Kurzbestätigungscode SVC verglichen und bei Übereinstimmung verifiziert. Das Eingabefeld 60 kann durch ein berührungsempfindliches Tastenfeld auf einem Touch-Display, welches der Benutzerführung und der Ausgabe des Verifikationsergebnisses dient, realisiert sein. In diesem Fall kann das Tastenfeld speziell für die Eingabe des SVC optimiert sein. Alternativ kann ein separates Display 80 vorgesehen sein.

Zusammenfassend wird durch die beschriebene Verwendung und Abfrage des Kurzbestätigungscode SVC auf überraschend einfache Weise eine Verwechslung beim Transport der Medikationscontainer MC von der Apotheke oder vom Lieferanten zum zugeordneten Patientenbett vermieden, ohne dass es umständlicher Scanvorgänge oder dergleichen bedarf.

FIG. 3 bis 10 zeigen verschiedene Varianten einer alterativen, noch weiter vereinfachten Implementierung des in FIG 1 und 2 beschriebenen Systemkonzepts.

Den Varianten ist gemeinsam, dass die Instanz des Infusion Order Manager (IOM) entfällt. Die jeweilige Infusionspumpe IP kommuniziert stattdessen (über die Zwischenstation "Backend") direkt mit dem EMR-System. Es handelt sich bevorzugt um eine bidirektionale Kommunikation, die über die pumpenseitigen Schnittstellen SS 1 und SS 4 und die EMR-seitigen Schnittstellen SS 2 und SS 3 initiiert wird (die hier im Beispiel getrennt gezeichnete und jeweils als Push-Schnittstelle beschriftete Hin- und Rückrichtung können auch anderweitig implementiert sein, beispielsweise als einzelne bidirektionale Leitung). Das heißt, das Backend spielt hier nur die Rolle einer Durchleitstation. Alternativ können die Infusionspumpen IP auch direkt mit dem EMR kommunizieren bzw. vom EMR angesprochen werden. Das heißt, das Backend kann aus dieser Sicht heraus sogar ganz entfallen bzw. von den Verbindungsleitungen oder vom Datenverkehr umgangen werden.

Ein weiterer Unterschied zum System und zum Verfahren aus FIG. 1 ist, dass die Infusionsaufträge (Infusion Orders) IO nun in einem Stapel, nämlich einem Infusion Order Backlog (IOB) im EMR zwischengespeichert und zum Abruf durch die bzw. zur Übermittlung an die Infusionspumpen IP bereit gehalten werden.

Bei der Variante gemäß FIG. 3 erfolgt die Patientenzuordnung via Bettplatz sowie eine automatische Verifikation im EMR-System. Die aktivierte Infusionspumpe IP übermittelt ihre Pumpen-ID, die ihr beispielsweise qua Anschluss oder Aufstellungsort zugeordnete Betten-ID und den vom User eingegebenen SVC an das EMR. Das EMR prüft darauf hin, ob es einen Infusionsauftrag gibt, der zu dem eingegebenen SVC passt, und ob der hinterlegte Bettplatz mit der von der Pumpe empfangenen Bettplatz-Zuordnung übereinstimmt. Im Ergebnis erfolgt dabei der folgende Check: Findet die anstehende Infusion tatsächlich an demjenigen Patienten statt, der im Infusionsauftrag IO spezifiziert ist? Falls nein, wird eine Fehlermeldung ausgegeben, falls ja wird die Infusionspumpe IP entsprechend dem übermittelten Infusionsauftrag IO programmiert bzw. eine bereits erfolgte Programmierung zur Ausführung freigegeben.

In der Variante gemäß FIG. 4 erfolgt eine Patientenzuordnung via Bettplatz, sowie eine automatische Verifikation der Bettplatz-Pumpen-Zuordnung im Infusionspumpen-Backend. Die aktivierte Infusionspumpe IP übermittelt dazu ihre Pumpen-ID und den vom User eingegebenen SVC einerseits an das EMR, andererseits an das Backend (die Übermittlung an das Backend kann auch indirekt von der Infusionspumpe IP via EMR erfolgen). Das EMR prüft darauf hin, ob es einen Infusionsauftrag IO gibt, der zu dem eingegebenen SVC passt, und das Backend prüft, ob der Bettplatz mit der von der Pumpe empfangenen Bettplatz-Zuordnung übereinstimmt.

In der Variante gemäß FIG. 5 erfolgt eine Patientenzuordnung via Bettplatz, sowie eine automatische Verifikation der Bettplatz-Pumpen-Zuordnung in der Infusionspumpe IP. Das heißt, die genannte Prüfung ist gegenüber der vorigen Variante in die Infusionspumpe IP selbst verlagert. Im Übrigen gilt das zuvor Gesagte.

In der Variante gemäß FIG. 6 erfolgt eine Patientenzuordnung via Bettplatz, im Unterschied zur vorigen Variante jedoch lediglich eine manuelle Verifikation an der Infusionspumpe IP, bei der der User manuell folgenden Check durchführt: Ist die Pumpe mit dem Patienten verbunden, der vom Infusionsauftrag IO angesprochen wird? Im Übrigen gilt das zuvor Gesagte.

Zusammenfassend beruhen die drei Varianten gemäß FIG. 3 bis 5 auf einer automatischen Verifikation der Bettplatz-basierten Patientenzuordnung an drei möglichen Stellen (EMR-System, Infusionspumpen-Backend, Infusionspumpe). In der Variante gemäß FIG. 6 hingegen erfolgt eine manuelle Verifikation der Bettplatz-basierten Patientenzuordnung. Die insgesamt vier Varianten beruhen also auf einer Bettplatz-basierten Patientenzuordnung.

Demgegenüber beruhen die vier Varianten gemäß FIG. 7 bis 10 auf einer Patienten-ID-basierten Patientenzuordnung. Die Patientenzuordnung über die Patienten-ID (in den Zeichnungen auch kurz P.ID) ist insgesamt deutlich besser/sicherer als die über den Bettplatz. Sie setzt jedoch voraus, dass die Patienten-ID in der Infusionspumpe IP vorhanden oder zumindest im Backend der jeweiligen Infusionspumpe IP zugeordnet ist.

In der Praxis ist eine Patienten-ID unter Umständen lang und unhandlich. Eine manuelle Eingabe an der Infusionspumpe IP könnte als umständlich oder sogar unzumutbar angesehen werden. Doch auch dieses Problem lässt sich über einen Short Code elegant lösen, konkret über einen Patienten-SVC, der beispielsweise auf einem vom Patienten getragenen Armband aufgedruckt, leicht ablesebar und leicht an der Infusionspumpe IP einzugeben ist. Das heißt konkret, eine aktuell für einen gewissen Zeitraum verwendete oder benutzte Untermenge aus einer Vielzahl länglicher Patienten-IDs wird in eineindeutiger Weise auf eine Menge wesentlich kürzerer und damit besser handhabbarer Patienten-SVCs abgebildet. Oder anders formuliert: Die (längliche) Patienten-ID wird dauerhaft genutzt und gespeichert. Nur der (kürzere) Patienten-SVC ist dem Patienten (bzw. der Patienten-ID) für einen gewissen Zeitraum zugeordnet und kann anschließend neu vergeben werden.

Unabhängig davon wird im Rahmen dieser Anmeldung nur vorausgesetzt, dass eine Zuordnung zwischen Infusionspumpe IP und Patienten-ID (und damit implizit auch dem Bettplatz) im System vorhanden, insbesondere im EMR hinterlegt ist.

Bei der Variante gemäß FIG. 7 erfolgt die Patientenzuordnung via Patienten-ID, sowie eine automatische Verifikation im EMR-System. Die aktivierte Infusionspumpe übermittelt ihre Pumpen-ID, die ihr zugeordnete oder bekannte Patienten-ID und den vom Benutzer eingegebenen SVC an das EMR. Das EMR prüft darauf hin, ob es einen Infusionsauftrag IO im Infusion Order Backlog IOB gibt, der zu dem eingegebenen SVC passt, und ob die Infusionspumpe IP demselben Patienten zugewiesen ist wie der Infusionsauftrag IO. Im Ergebnis erfolgt dabei der folgende Check: Findet die anstehende Infusion tatsächlich an demjenigen Patienten statt, der im Infusionsauftrag IO spezifiziert ist? Falls nein, wird eine Fehlermeldung ausgegeben, falls ja wird die Infusionspumpe IP entsprechend dem übermittelten Infusionsauftrag IO programmiert bzw. eine bereits erfolgte Programmierung zur Ausführung freigegeben.

In der Variante gemäß FIG. 8 erfolgt die Patientenzuordnung via Patienten-ID, sowie eine automatische Verifikation der Patienten-Pumpen-Zuordnung im Infusionspumpen-Backend. Das heißt, die genannte Prüfung ist gegenüber der vorigen Variante in das Backend verlagert. Die aktivierte Infusionspumpe IP übermittelt dazu ihre Pumpen-ID, die ihr zugeordnete oder bekannte Patienten-ID und den vom Benutzer eingegebenen SVC einerseits an das EMR, andererseits an das Backend (die Übermittlung an das Backend kann auch indirekt von der Infusionspumpe IP via EMR erfolgen). Im Übrigen gilt das zuvor Gesagte.

In der Variante gemäß FIG. 9 erfolgt eine Patientenzuordnung via Patienten-ID, sowie eine automatische Verifikation der Patienten-Pumpen-Zuordnung-Zuordnung in der Infusionspumpe IP. Das heißt, die genannte Prüfung ist gegenüber der vorigen Variante in die Infusionspumpe selbst verlagert. Die Infusionspumpe IP muss dann an das EMR nur ihre Pumpen-ID und den eingegebenen SVC übermitteln. Im Übrigen gilt das zuvor Gesagte.

In der Variante gemäß FIG. 10 erfolgt eine Patientenzuordnung via Patienten-ID, im Unterschied zur vorigen Variante jedoch lediglich eine manuelle Verifikation an der Infusionspumpe IP, bei der der User manuell folgenden Check durchführt: Ist die Infusionspumpe IP mit dem Patienten verbunden, der vom Infusionsauftrag IO angesprochen wird? Im Übrigen gilt das zuvor Gesagte.

### Bezugszeichenliste

- APO: Apotheke
- Backend: Backend
- Bett: Patientenbett
- EMR: elektronisches Patientendaten Management-System
- IO: Infusionsauftrag
- IOB: Infusionsauftrags-Backlog
- IOM: Infusionsauftragsmanager
- IP: Infusionspumpe
- IPS: Infusionspumpensystem
- MC: Medikationscontainer
- ME: Medizinische Einrichtung
- MO: Medikationsauftrag
- P.ID: Patienten-ID
- PK: Pflegekraft
- SVC: Kurzbestätigungscode
- SS: Schnittstelle
- Z: Zuordnung

- 10: Aufnahme
- 20: Pumpe
- 30: Steuerung
- 40: Speicher
- 50: Schnittstellenmodul
- 60: Eingabefeld
- 70: Verifikationseinheit
- 80: Display

## Patentansprüche

1. Verfahren zur automatischen Fernprogrammierung von Infusionspumpen (IP) mittels Infusionsaufträgen (IO), die in/von einem Patientendaten Management-System (EMR) einer medizinischen Einrichtung (ME) bereitgestellt werden, wobei die medizinische Einrichtung (ME)
• eine Anzahl von Betten aufweist, die jeweils mit einem Patienten belegbar sind,
• eine Anzahl von Infusionspumpen (IP) aufweist,
wobei
• das Patientendaten Management-System (EMR) in einem Infusionsauftrags-Backlog (IOB) Infusionsaufträge (IO) zwischenspeichert,
• die jeweilige Infusionspumpe (IP) über eine Anzahl von elektronischen Schnittstellen (SS 1, SS 2, SS3, SS 4) mit dem Infusionsauftrags-Backlog (IOB) kommuniziert und zwischengespeicherte Infusionsaufträge (IO) abruft oder empfängt,
wobei ferner
• dem jeweiligen Infusionsauftrag (IO) ein von einem Menschen lesbarer Kurzbestätigungscode (SVC) als eindeutige Kennung unmittelbar zugeordnet und bei Übermittlungen mitgegeben wird,
• dem jeweiligen Infusionsauftrag (IO) ein Medikationscontainer (MC) zugeordnet wird, der mit einem den zugeordneten Kurzbestätigungscode (SVC) enthaltenden Informationsträger ausgestattet oder verbunden ist,
• die jeweilige Infusionspumpe (IP) eine Aufnahme (10) oder einen Anschluss für einen der Medikationscontainer (MC) aufweist,
• der jeweiligen Infusionspumpe (IP) ein Eingabemittel, insbesondere ein an der Infusionspumpe (IP) angebrachtes Eingabefeld (60), zur Eingabe des zugehörigen, dem Informationsträger des Medikationscontainers (MC) entnehmbaren Kurzbestätigungscodes (SVC) zugeordnet ist,
wobei schließlich
in einem Verifikationsschritt ein am Eingabemittel eingegebener Kurzbestätigungscode (SVC) mit dem Kurzbestätigungscode (SVC), der dem von der Infusionspumpe (IP) empfangenen Infusionsauftrag (IO) unmittelbar zugeordnet ist, verglichen wird, und wobei nur bei Übereinstimmung der Kurzbestätigungscodes (SVC) der Infusionsauftrag (IO) an die Infusionspumpe (IP) übertragen und von dieser ausgeführt wird oder auf Aufforderung ausführbar ist.

2. Verfahren nach Anspruch 1, wobei eine Zuordnung zwischen Patient und Infusionspumpe (IP) indirekt über die Bettplatzzuordnung der Infusionspumpe (IP) erfolgt.

3. Verfahren nach Anspruch 1, wobei eine Zuordnung zwischen Patient und Infusionspumpe (IP) über eine der Infusionspumpe (IP) bekannte oder an ihr einzugebende Patienten-ID (P.ID) erfolgt.

4. Verfahren nach Anspruch 3 oder 4, wobei die Zuordnung zwischen Patient und Infusionspumpe (IP) anhand des dem Kurzbestätigungscode (SVC) zugeordneten Infusionsauftrags (IO) automatisch verifiziert wird.

5. Verfahren nach Anspruch 4, wobei die Verifikation im Patientendaten Management-System (EMR), in der Infusionspumpe (IP) oder in einem Backend eines Infusionspumpensystems (IPS) erfolgt.

6. Verfahren nach einem der vorangehenden Ansprüche, wobei dem jeweiligen Infusionsauftrag (IO) ein Gültigkeitszeitraum oder eine Gültigkeitsdauer zugeordnet und im Verifikationsschritt berücksichtigt wird.

7. Verfahren nach einem der vorangehenden Ansprüche, wobei der Kurzbestätigungsode (SVC) ein alphanumerischer Code ist.

8. Verfahren nach einem der vorangehenden Ansprüche, wobei der der Kurzbestätigungsode (SVC) zwei, drei- oder vierstellig ist.

9. Verfahren nach einem der vorangehenden Ansprüche, wobei der der Medikationscontainer (MC) mit dem zugeordneten Kurzbestätigungscode (SVC) beschriftet oder gekennzeichnet ist.

10. System zur automatischen Fernprogrammierung von Infusionspumpen (IP) mittels Infusionsaufträgen (IO), die in einem Patientendaten Management-System (EMR) einer medizinischen Einrichtung (ME) bereitgestellt werden, wobei die medizinische Einrichtung (ME)
• eine Anzahl von Betten aufweist, die jeweils mit einem Patienten belegbar sind,
• eine Anzahl von Infusionspumpen (IP) aufweist,
wobei
• das Patientendaten Management-System (EMR) in einem Infusionsauftrags-Backlog (IOB) Infusionsaufträge (IO) zwischenspeichert,
• die jeweilige Infusionspumpe (IP) über eine Anzahl von elektronischen Schnittstellen (SS 1, SS 2, SS3, SS 4) mit dem Infusionsauftrags-Backlog (IOB) kommuniziert und zwischengespeicherte Infusionsaufträge (IO) abruft oder empfängt,
wobei ferner
• dem jeweiligen Infusionsauftrag (IO) ein von einem Menschen lesbarer Kurzbestätigungscode (SVC) als eindeutige Kennung unmittelbar zugeordnet ist,
• dem jeweiligen Infusionsauftrag (IO) ein Medikationscontainer (MC) zugeordnet ist, der mit einem den zugeordneten Kurzbestätigungscode (SVC) enthaltenden Informationsträger ausgestattet oder verbunden ist,
• die jeweilige Infusionspumpe (IP) eine Aufnahme (10) oder einen Anschluss für einen der Medikationscontainer (MC) aufweist,
• der jeweiligen Infusionspumpe (IP) ein Eingabemittel, insbesondere ein an der Infusionspumpe (IP) angebrachtes Eingabefeld (60), zur Eingabe des zugehörigen, dem Informationsträger des Medikationscontainers (MC) entnehmbaren Kurzbestätigungscodes (SVC) zugeordnet ist,
wobei schließlich
das System eine Verifikationseinheit (70) umfasst, die dazu ausgelegt oder programmiert ist, einen am Eingabemittel eingegebenen Kurzbestätigungscode (SVC) mit dem Kurzbestätigungscode (SVC), der dem von der Infusionspumpe (IP) empfangenen Infusionsauftrag (SVC) unmittelbar zugeordnet ist, zu vergleichen, so dass nur bei Übereinstimmung der Kurzbestätigungscodes (SVC) der Infusionsauftrag (IO) an die Infusionspumpe (IP) übertragen und von dieser ausgeführt wird oder auf Aufforderung ausführbar ist.

11. Infusionspumpe (IP) zur Verwendung in einem System nach Anspruch 10, mit
• einer Aufnahme (10) oder einem Anschluss für einen Medikationscontainer (MC),
• einem Eingabemittel, insbesondere einem Eingabefeld (60) zur Eingabe eines von einem Informationsträger des Medikationscontainers (MC) entnehmbaren Kurzbestätigungscodes (SVC),
• einer Anzahl von elektronischen Schnittstellen (SS 1, SS 4), die mit einem Infusionsauftrags-Backlog (IOB) eines Patientendaten Management-Systems (EMR) kommunizieren und von dort zwischengespeicherte Infusionsaufträge (IO) abrufen oder empfangen.

12. Infusionspumpe (IP), welche eine eine Verifikationseinheit (70) umfasst, die dazu ausgelegt oder programmiert ist, einen am Eingabemittel eingegebenen Kurzbestätigungscode (SVC) mit dem Kurzbestätigungscode (SVC), der dem von der Infusionspumpe (IP) empfangenen Infusionsauftrag (SVC) unmittelbar zugeordnet ist, zu vergleichen, so dass nur bei Übereinstimmung der Kurzbestätigungscodes (SVC) der Infusionsauftrag (IO) an die Infusionspumpe (IP) übertragen und von dieser ausgeführt wird oder auf Aufforderung ausführbar ist
